# EUROPEAN PATENT SPECIFICATION

(11) **EP 1 109 573 B1**
(45) Date of publication and mention of the grant of the patent: **05.06.2002**
(21) Application number: 99940446.0
(22) Date of filing: 30.08.1999
(51) Int. Cl.: A61K 38/45, A61P 25/00

(54) **PHARMACEUTICAL COMPOSITIONS COMPRISING FACTOR XIIIa FOR NERVE HEALING**
PHARMAZEUTISCHE ZUSAMMENSETZUNG ENTHALTEND FACTOR XIIIA FÜR NERVENHEILUNG
COMPOSITIONS PHARMACEUTIQUES COMPRENANT LE FACTEUR XIIIa POUR LA REGENERATION NERVEUSE

(30) Priority: 30.08.1998 IL 12598398
(43) Date of publication of application: 27.06.2001
(73) Proprietor: YEDA RESEARCH & DEVELOPMENT COMPANY, LTD., 76100 Rehovot (IL)
(72) Inventor: SCHWARTZ-EISENBACH, Michal, 76353 Rehovot (IL); MONSONEGO, Alon, 73112 Kfar Hanoar Ben-Shemen (IL); MIZRAHI, Tal, 76885 Moshav Ge'Alya 21 (IL)
(74) Representative: VOSSIUS & PARTNER
(86) International application number: IL9900472
(87) International publication number: WO0012119

(56) References cited:
- WO-A-94/03059
- WO-A-94/22470
- WO-A-97/09885
- GREENBERG CS: "Transglutaminases: multifunctional cross-linking enzymes that stabilize tissues" THE FASEB JOURNAL, vol. 5, December 1991 (1991-12), pages 3071-3077, XP002131496 cited in the application
- ADANY R: "Intracellular Factor XIII: Cellular Distribution of Factor XIII Subunit a in Humans" SEMIN THROMB HEMOST, vol. 22, no. 5, 1996, pages 399-408, XP000876863 cited in the application

## Description

### FIELD AND BACKGROUND OF THE INVENTION

The present invention relates to the use of pharmaceutical compositions comprising as active ingredient Factor XIIIa for nerve healing.

Intensive research over the last two decades has been aimed at uncovering the reasons why the mammalian central nervous system (CNS) fails to regenerate after white matter lesion, while lesions of the CNS in lower vertebrates (e.g. fish) or of the peripheral nervous system (PNS) of mammals are followed by successful recovery. It is well known that the environment of the injured CNS is inhibitory to regrowth (1, 2), and that the degeneration of injured axons triggers processes leading to secondary degeneration of neurons that escaped the initial injury (3, 4). Modulation of the CNS environment was shown to result in axonal regrowth both *in vitro* and *in vivo* (5-8), confirming that the inability of the mammalian CNS to achieve functional recovery after injury is not necessarily a reflection of an intrinsic property of the neurons themselves.

A prerequisite for the recovery of any injured tissue is effective dialogue between the damaged tissue and the immune system (9, 10). A growing body of evidence indicates that the interaction between the immune system and the CNS is restricted under normal physiological conditions and that this restriction is manifested from the earliest stages following CNS injury (11). No such restriction appears to exist in the peripheral nervous system (PNS) of mammals or in the CNS of lower vertebrates.

In line with the above findings are our observations that regeneration in the fish CNS is correlated with the activity of inflammatory cells and their products (12). Among the activities thought to be associated with inflammation is an enzyme of the transglutaminase (TGase) family (13).

Blood coagulation factor XIII (hereinafter FXIII), also known as fibrin-stabilizing factor, fibrinase and Laki-Lorand factor, is found in the plasma as a tetrameric protein consisting of two types of subunits (a₂b₂-subunits), of which the a-subunits (a₂) are potentially active (hereinafter FXIIIa). FXIII is found also in platelets, monocytes, and monocyte-derived macrophages. The cellular FXIII lacks the b-subunits and is a homodimer (a₂) of the a-subunits.

Plasma FXIII circulates in association with its substrate precursor, fibrinogen. During the last stage of the coagulation process, prothrombin is activated by its cleavage to thrombin, which catalyzes the cleavage of both fibrinogen and FXIIIa, making them more susceptible for complex formation. The dissociation of the a- and b-subunits of FXIII leads to the exposure of the originally buried active site on the free a-subunits. Subsequently, the cleaved a₂-subunit is dissociated from the dimer b₂ to form the active FXIIIa enzyme, which catalyzes the cross-linking of fibrin in the presence of Ca²⁺. The a-subunit of FXIIIa belongs to a family consisting of several genes which encode for enzymes with a common active-site structure, well-conserved among species (17).

FXIII is well known for its role in blood clotting (14-16). Deficiency of FXIII produces a clinical hemorrhagic diathesis. FXIII is an essential component of fibrin sealant used clinically in surgical procedures for wound healing and tissue repair. Accumulating evidence points to an additional role for FXIIIa in proliferation of connective tissues via its expression by invading macrophages (18, 19).

### SUMMARY OF THE INVENTION

According to the present invention, it was found that some characteristics of the a-subunit of FXIII in fish plasma are distinct from those of its mammalian counterpart. The 55-kDa form in which the FXIIIa enzyme is constitutively expressed in fish plasma and nerve tissue indicates states of activation and regulation of expression different from those of the 80-kDa form in which FXIIIa is expressed in mammals. Injury-induced variations in the a-subunit of FXIIIa expression in regenerating and in non-regenerating nerve tissues were also investigated.

The present invention relates to the use of Factor XIIIa for the preparation of a pharmaceutical composition useful for nerve healing. The FXIIIa may be the commercially available natural enzyme purified from human plasma or recombinant FXIIIa.

The pharmaceutical composition may be presented in any suitable form for administration of factor XIIIa, preferably in the form of a sterile freeze-dried powder suspended in a sterile pharmaceutically acceptable carrier such as water or phosphate-buffered saline (PBS), for injection locally at a site of injury or disease. The amount of FXIIIa to be administered will depend on the type of injury or disease and the age and condition of the patient as to be determined by a skilled physician, but preferably it will be in the range of 0.1 - 1U/kg body weight.

### DESCRIPTION OF THE DRAWINGS

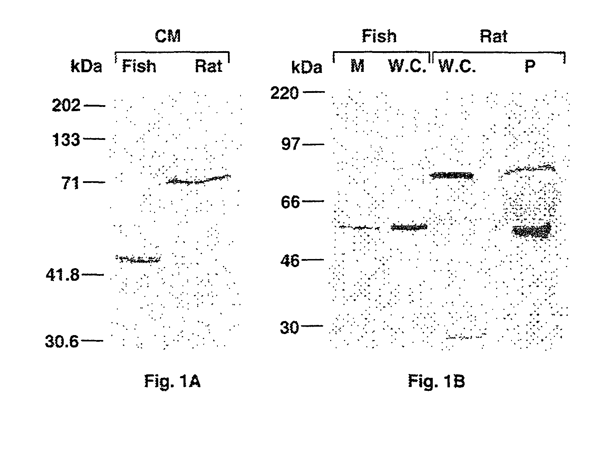

**Figs. 1A-1B.** Western blot analysis of FXIIIa in fish and rat. 1A. Fish optic nerve and rat sciatic nerve were incubated in medium for 1.5 h. The collected conditioned media were analyzed by SDS-PAGE and tested for immunoreactivity with rabbit polyclonal antibodies raised against the a-subunit of human FXIIIa. **1B.** Immunoreactivity of the a-subunit of FXIIIa in fish white blood cells (W.C.) and in monocytes (M) was compared with that in rat white cells and plasma. Note the 55-kDa protein detected in fish versus the 80-kDa protein in rat. The cleavage product (55-kDa protein) of rat plasma FXIIIa is similar in size to the protein detected in fish.

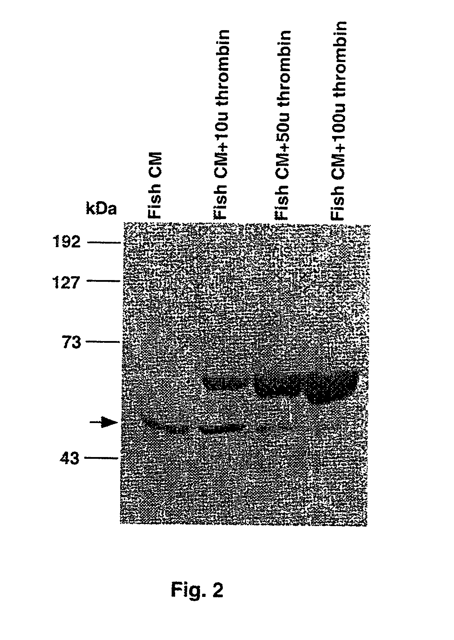

**Fig. 2.** Loss of the a-subunit of FXIIIa immunoreactivity and precipitate formation in fish optic nerve-conditioned medium upon the gradual addition of thrombin. Fish optic nerve-conditioned medium was prepared as described. Conditioned media were subjected to complex formation in the presence of increasing amounts of human thrombin (Omrix Biopharmaceutical, Brussels, Belgium) for 1 h at room temperature, followed by centrifugation for 15 min at 4°C. Supernatants were collected and subjected to SDS-PAGE, followed by Western blot analysis using anti a-subunit antibodies. Note the decrease in the level of 55-kDa immunoreactive protein in the presence of high thrombin concentrations. The higher band is not a result of immunoreactivity but of human thrombin overload, since it also appears in Ponceau staining of the blot in the absence of conditioned medium (data not shown).

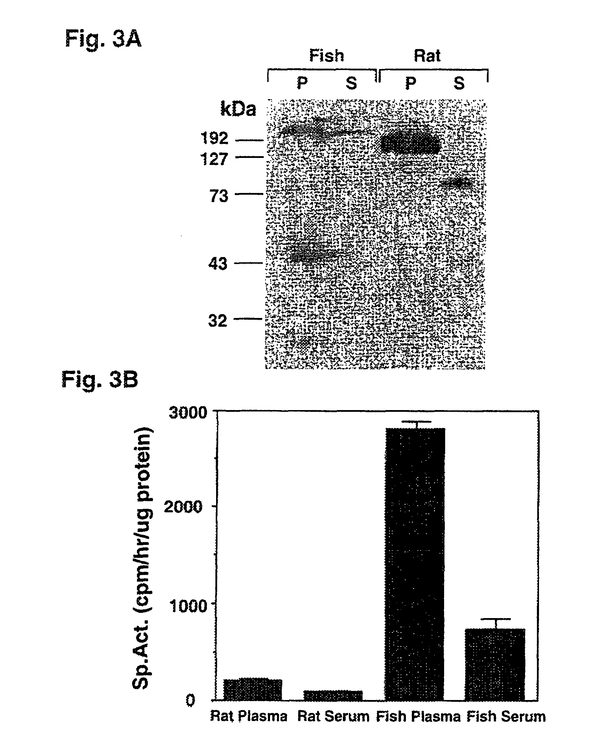

**Figs. 3A-3B.** Immunoreactivity of the a-subunit of FXIIIa and cross-linking activity in rat and fish plasma. Plasma (P) and serum (S) samples from fish and rats, obtained as described in Materials and Methods, were analyzed for the presence of the a-subunit of FXIIIa-immunoreactivity by Western blotting following SDS-PAGE under nonreducing conditions (**3A**). FXIIIa cross-linking activity in plasma and serum was measured by incorporation of [³H] putrescine into N,N-dimethylated casein **(3B).**

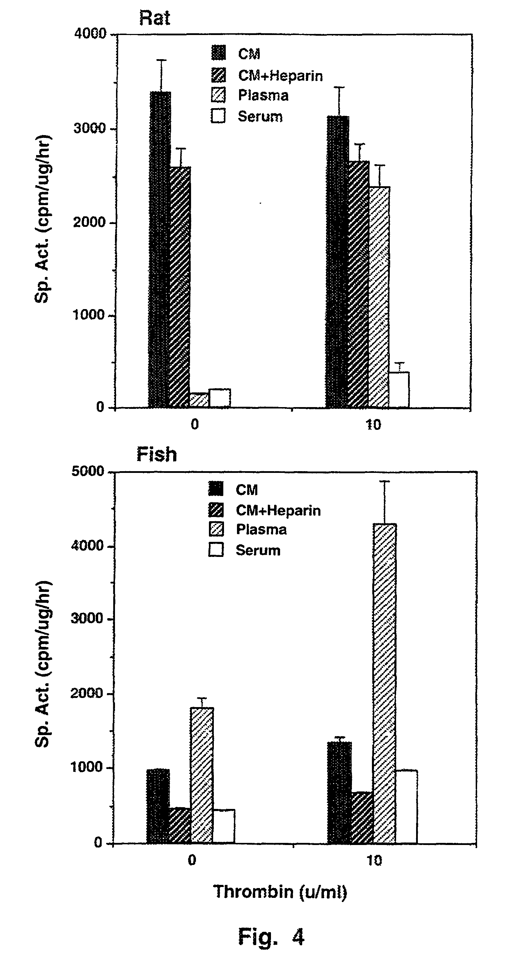

**Fig. 4.** Cross-linking activity of plasma and nerve-conditioned media as a function of thrombin concentration. Media conditioned by fish optic nerve or rat sciatic nerve with or without heparin, as well as fish or rat plasma and serum, were analyzed for their cross-linking activities in the absence or presence of thrombin (10 U/ml). Note the low cross-linking activity of rat plasma relative to fish plasma in the absence of exogenous thrombin.

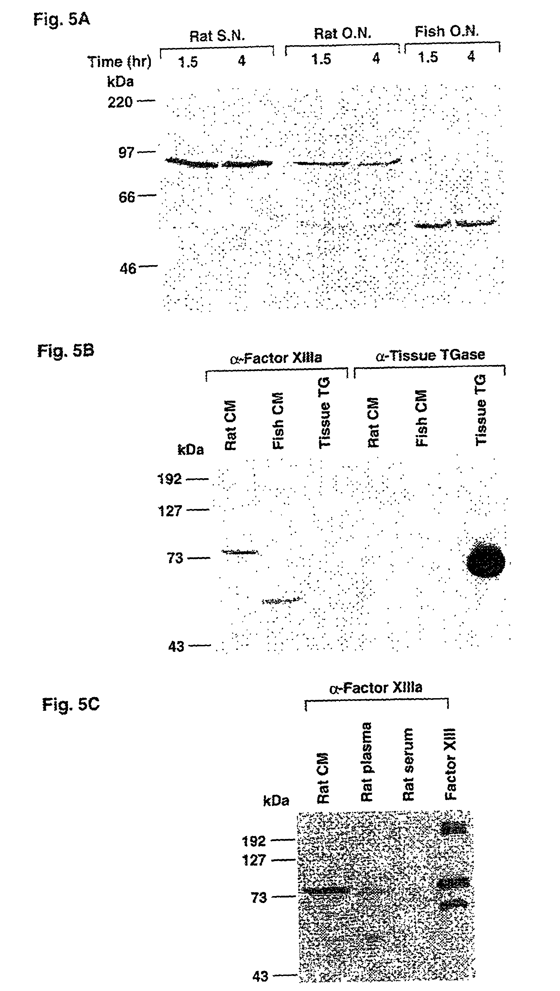

**Figs. 5A-5C.** Characterization of rat nerve FXIIIa. The a-subunit of FXIIIa immunoreactivity in the various nerve preparations, as a function of time incubation of the nerve in the conditioned media preparation, is shown in panel (5A). Fish optic nerve-conditioned medium, rat optic nerve-conditioned medium and purified rat tissue-type TGase were subjected to SDS-PAGE under reducing conditions, and to Western Blot analysis using rabbit polyclonal antibodies raised against the a-subunit of FXIII or rabbit affinity-purified polyclonal antibodies directed against rat tissue-type TGase **(5B).** To confirm the specificity of the antibodies directed against FXIIIa, we also analyzed rat plasma and serum and Fibrogammin-P as human plasma FXIII (Behring, Marburg, Germany) under the same experimental conditions **(5C).**

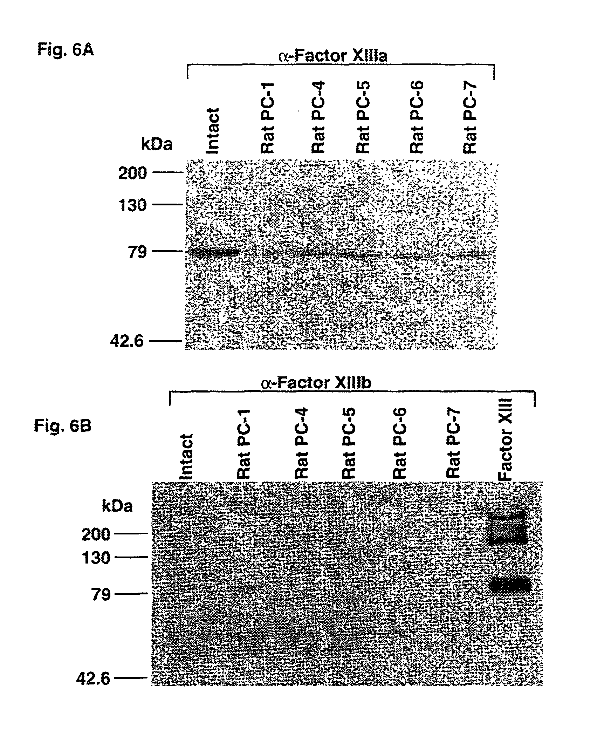

**Figs. 6A-6B.** Immunoreactivity of the a and b subunits of FXIII in intact and injured rat sciatic nerve. Rat sciatic nerves were crushed and excised on different days after the injury. Medium conditioned by intact or injured (post-crush, PC) nerves was analyzed by SDS-PAGE, and immunoreactivities for the a-subunit (**6A**) and b-subunit **(6B)** were assayed. Note that levels of the a-subunit are highest in the intact nerve, whereas the b-subunit was detected mainly on day 1 after injury. Fibrogammin-P as human plasma FXIII was used as a positive control.

**Figs. 7A-7B.** Comparative analysis of FXIIIa cross-linking activity in medium conditioned by nerves of rat or fish following axonal injury. Rat sciatic and optic nerves and fish optic nerve were crushed and excised on different days after the injury. Media conditioned by these nerves were analyzed for cross-linking activity as described. **7A.** Rat sciatic and optic nerve. **7B.** Fish optic nerve.

### DETAILED DESCRIPTION OF THE INVENTION

Recent findings have led to changes in the traditional concept of nerve recovery, including the realization that injured nerves, like any other injured tissue, need the assistance of blood-derived cells and factors in order to heal.

In the present application, we show that factor XIIIa (FXIIIa, the potentially active a₂-subunit of factor XIII), an enzyme that participates in blood coagulation by stabilizing the fibrin clot, is also active in the nervous system, where it plays a key role in the healing of injured tissue. It is shown herein that the plasma, macrophages and nerves of fish contain a 55-kDa form of transglutaminase (TGase) that cross-reacts immunologically with the a-subunit of FXIII in mammals (80 kDa). The fish enzyme in the plasma, unlike its mammalian counterpart, is active, pointing to a difference in control of the coagulation pathway in the two species. Analysis of FXIIIa expression in mammalian neural tissues and their response to injury revealed high levels of the enzyme in media conditioned by peripheral nerves as compared with medium conditioned by nerves of the central nervous system. Furthermore, similarity was observed in the postinjury behavior of FXIIIa in regenerating nerve tissues (peripheral nervous system of mammals and the central nervous system of fish). This indicates that the postinjury level of factor XIIIa in the nervous system may be related to the tissue's regenerative capacity, and that FXIIIa may therefore be a link underlying a possible association between the processes of blood coagulation and nerve healing.

Until very recently the CNS was viewed as a unique tissue, and the rules governing healing of tissues in general were thought not to be applicable to the CNS. Growing understanding of the processes involved has led to a change in this concept. For example, an accumulating body of information suggests that components of the inflammatory response and of blood coagulation may play as vital a part in CNS healing as in the healing of other tissues. According to the present invention, we demonstrate that FXIIIa, a component of the coagulation process, is present in nervous tissue, and we provide evidence for a correlation between the post-injury appearance and activation of the enzyme and the regenerative ability of the tissue. We also show that the activation of coagulation pathways and wound-healing factors is regulated differently in fish and rats.

FXIII is an inactive tetrameric complex consisting of a₂ and b₂ subunits, which probably associate in the circulating blood. Levels of b₂ subunits are higher than those of a₂ subunits, thus preventing the formation of free FXIIIa susceptible to activation by Ca²⁺ and/or thrombin. Thrombin, a cleavage product of prothrombin, is considered to play a central regulatory role in blood clot formation through concomitant activation of fibrinogen and of FXIIIa. We show here that FXIIIa is indeed inactive in rat plasma, unless it is activated by thrombin to initiate the process of complex formation. In contrast, fish FXIIIa appears to be active not only in the presence but also in the absence of exogenous thrombin.

The dominant a-subunit of FXIIIa immunoreactive protein in fish was found to have a molecular size of 55-kDa. Its presence in fish monocytes, white blood cells and plasma is similar to that reported for the a-subunit of FXIIIa in mammals (16, 20, 24). The low molecular weight of the enzyme in fish seems to reflect, at least partially, the state of activation of the blood-clotting pathway in the fish relative to that in mammals. It thus seems that fish express constitutively a low molecular weight form of FXIIIa, which is in a higher state of activation than the 80-kDa mammalian FXIIIa, needed for cross-linking activity. However, as shown in the present application, the fish enzyme can be further activated by thrombin. This phenomenon might be due to the nature of the regulatory mechanism in fish. Clot formation *in vitro* indeed occurs more rapidly in fish blood than in the blood of mammals. It is also possible that other members of the TGase family contribute to cross-linking activity in the plasma. If so, high levels of cross-linking activity would also be expected in the serum, which is not the case. The high state of activation of the blood coagulation protein in fish may reflect the evolutionary transition from an open to a closed blood system in vertebrates or adaptation to environmental conditions. A disadvantage of such response regulation in fish is that it is not limited to the region of the injury, but is systemic, and therefore there is increased susceptibility for random clot formation, a process which is fatal.

In addition to its participation in blood coagulation, FXIIIa appears to be the extracellular TGase involved in wound healing (16, 19, 20). The mammalian peripheral nervous system (PNS) tissue, according to the present invention, seems to respond very efficiently to injury, as shown for example by the fact that FXIIIa becomes fully active (i.e. could not be further activated by the addition of thrombin) as a result of sciatic nerve excision. This finding is in line with other studies that point to the presence and activation of thrombin in the mammalian nervous system in response to injury. At injury sites, thrombin regulates gene expression and outgrowth from neurons and astrocytes (25, 26). However, high concentrations of thrombin were shown to be toxic for both astrocytes and neurons (27).

The cellular source of the FXIIIa found in nervous tissue may be macrophages resident in normal nerve tissue (i.e. microglia) and/or macrophages invading after injury. The lack of correlation between the levels of the a₂-subunit and the b₂-subunit during wound healing in rat PNS suggests that the enzyme found in the tissue conditioned media is only partially derived from the blood, and that some of it may be a local product of invading or resident cells or that the a-subunit undergoes extensive consumption immediately after injury.

Rat optic nerve, as part of the mammalian central nervous system (CNS), fails to regenerate after injury, and axons that may have escaped the primary lesion ultimately fall victim to the hostile environment formed by the degenerating axons. In earlier work, we showed that a 55-kDa enzyme belonging to the TGase family is present in regenerating fish nerve and plays a key role in recovery when injected into transected optic nerve of mammals (29). Nerve FXIIIa might play a role in cross-linking of fibrin and other ECM components (14, 16, 17), thereby stabilizing the extracellular matrix and making it more effective in creating local concentrations of trophic factors, growth factors and cytokines. Nerve TGase might be effective in cross-linking cytokines and growth factors.

The invention will now be illustrated by the following non-limiting Examples.

### EXAMPLES

### MATERIALS AND METHODS

**Preparation of nerve conditioned medium.** Carps (*Cyprinus carpio,* 800-1200g) were anesthetized with 0.05% 3-aminobenzoic acid ethyl ester (Sigma, Israel), and rats (Wistar, 8-week old males) were anesthetized with 0.2 ml ketamine (Rhone Merieux) mixed with 0.2 ml xylazine 2% (Vitamed, Israel). Optic nerves from fish and optic and sciatic nerves from rats were crushed (30 sec) with forceps. The nerves were excised at different times after injury and incubated in serum-free medium for 1.5 hr at room temperature. The resulting conditioned media were collected, centrifuged at 15,800xg for 5 min to remove tissue fragments, and the supernatants were collected and stored at -20°C.

**Plasma preparation.** Rat peripheral blood was withdrawn from the heart into a 10-ml syringe coated with heparin (5000 u/ml, Calbiochem, La Jolla, CA, USA) and containing 100 µl heparin. In fish the carotid artery was cut and peripheral blood was collected from the eye cavity, using a pasteur pipette coated with heparin (10000 u/ml), into 2 ml tubes containing 2-3 drops of heparin. The tubes were placed on ice, then centrifuged at 15,800xg for 15 min at 4°C, and the supernatants were collected. **Serum preparation.** Serum was prepared by the procedure described for plasma, but without heparin. The samples were incubated at room temperature for 15 min in (fish) and 30 min (rat), placed on ice same lengths of time, centrifuged at 15,800xg for 15 min at 4°C, and the supernatants were collected.

**White blood cell purification.** Peripheral blood mononuclear cells were obtained by one-step Percoll (Pharmacia) gradient fractionation. Blood was withdrawn as described for plasma preparation, diluted 1:1 with warm (37°C) phosphate-buffered saline (PBS), incubated for 5 min at room temperature and then subjected to Percoll fractionation (1.077 g/ml) (Pharmacia, Sweden). The Percoll-blood mixture was centrifuged in a rotating Sorvall centrifuge at 800xg 25-30°C for 25 min. The monocyte-enriched fraction was isolated from the interphase and washed twice with PBS to remove Percoll traces. For preparation of white blood cell homogenates the cells were collected by centrifugation, resuspended in extraction buffer (10 mM Tris PH 7.5, 150 mM NaCl, 1% Triton, 1 mM EDTA pH 8.0, and protease inhibitors) and incubated for 2 h at 4°C. After centrifugation for 5 min at 15,800xg at 4°C, the supernatant was collected. For preparation of monocyte homogenates the cells were resuspended in L15 medium and incubated in 3-cm petri dish for 1 h at room temperature. The adherent cells were washed several times with PBS and incubated with 500 µl of cells extraction buffer for 2 h at 4°C. After centrifugation for 5 min at 15,800xg at 4°C, the supernatants were collected and stored frozen.

**TGase activity assay.** TGase activity was assayed by measuring incorporation of putrescine into N,N-dimethylated casein. The reaction mixture contained 50 mM Tris-HCl, pH 8.0 or pH 9.0 for rat and fish CM respectively, 5 mM DTT, 5 mM CaCl₂, 0.075 µM putrescine [³H] (38.7 Ci/mmole) (DuPont NEN) and 4 mg/ml N,N-dimethylated casein (Sigma). The reaction mixture was incubated for 0.5 h or 1 h at 37°C, and then placed on ice. Cold trichloroacetic acid (TCA) was added to a final concentration of 5% for 15 min. Samples were centrifuged (14000xg for 5 min at room temperature) and the pellet was washed twice with 1 ml of 5% TCA and once with 100% ethanol. Samples were dried and resuspended in 200 µl of 0.1 N NaOH. Radioactivity was measured in 10 ml of scintillation liquid (40% lumax, 60% xylene). All the activity assays were done at least three times, each experiment in triplicate. Each activity is presented by average ± SEM of one experiment.

**Western blot analysis.** The various preparations (detailed in the Examples) were subjected to SDS-PAGE using 10% acrylamide slab gels. Following electrophoresis, proteins were transferred to a nitrocellulose membrane for 2.5 h at 200 mA (in Tris-glycine). The immune reaction was carried out as follows: the blot was incubated overnight in PBS containing 5% slim milk at 4°C and then with the antibodies (diluted 1:1000) in PBS containing 5% slim milk for 2 h at 37°C. This was followed by several washings with PBS containing 0.05% Tween-20, incubation with 1:10000 alkaline phosphatase-conjugated goat anti-rabbit IgG (Jackson) for 1 h at room temperature, several washings with PBS containing 0.05% Tween-20, and development with an ECL detection system (Amersham) for 1 min. Each Western blot analysis was repeated at least three times.

**Antibodies:** For immunodetection of the a-subunit protein we have used rabbit polyclonal antibodies directed to human a-subunit of FXIII (Centeon Pharma GmbH, Marburg, Germany) (20); For immunodetection of b-subunit we have used rabbit polyclonal antibodies directed to human b-subunit of FXIII (Calbiochem).

### Example 1

A 51-kDa protein was shown to be an active cleavage product of the a-subunit of human FXIIIa *in vitro* (21). To test the possibility that a 55-kDa protein purified from regenerating fish optic nerve and suggested to be a member of the TGase family (13, PCT Publication WO 94/03059), is a form of a-subunit of FXIIIa, we analyzed fish optic nerve-conditioned medium for its reactivity with antibodies directed to the a-subunit of FXIIIa.

As shown in Fig. 1A, Western blot analysis of the fish optic nerve-conditioned medium showed a single immunoreactive band of 55 kDa rather than the expected 80 kDa protein in rat sciatic nerve-conditioned medium. Monocytes and platelets, which are potential sources of FXIIIa in plasma and wounded tissue (20, 22, 23), were also tested for the presence of this 55 kDa immunoreactive protein. Western blot analysis revealed the presence of this 55 kDa immunoreactive proteins in extracts derived from both fish blood monocytes and white blood cells (Fig. 1B). No immunoreactive signal was obtained when the fish optic nerve-conditioned medium was subjected to Western blot analysis using rabbit polyclonal antibodies raised against fish tissue-type TGase (data not shown), further suggesting that the 55 kDa protein is the dominant form of TGase enzyme in the fish optic nerve-conditioned medium. Under the same experimental conditions, rat white blood cells and plasma exhibited, as expected, the known 80 kDa a-subunit of FXIIIa. In rat plasma we also detected a 55 kDa protein that immunoreacted with the antibodies against the a-subunit, probably as a result of a second cleavage by thrombin.

### Example 2

To find out whether the 55 kDa protein in the fish conditioned media is indeed involved in clot formation and therefore is a cross-linking enzyme, we examined its amount following addition of thrombin and consequent precipitate formation. Addition of increasing amounts of thrombin to the conditioned media resulted in a gradual decrease in the amount of the 55 kDa immunoreactive protein in the supernatant (Fig. 2), with the concomitant appearance of visible precipitates. The findings point to the formation of a precipitable complex involving the 55 kDa protein. This situation is reminiscent of plasma coagulation, where the resulting serum is devoid of coagulating components. These results thus support the suggestion that the 55 kDa is a form of a-subunit of FXIIIa and is potentially activated by thrombin for cross-linking activity.

### Example 3

To further examine the possibility that the 55 kDa protein found in fish optic nerve-conditioned medium is the form in which FXIIIa exists in fish plasma (rather than the 80 kDa form common in other species), we compared the a-subunit, detected by immunoreactivity, in the plasma and the serum. SDS-PAGE analysis, in the absence of boiling and reducing conditions, followed by Western blot analysis (Fig 3A), showed that a shorter form of the a-subunit of FXIIIa immunoreactive protein, of molecular weight similar to that found in the fish optic nerve preparation, is also present in fish plasma. Under non-reducing conditions, the molecular size of the complex in the fish is ∼240 kDa, rather than 320 kDa as in the rat. As expected, the amounts of both the a-subunit and the complex (probably consisting a₂b₂ subunits) were markedly decreased in the fish serum samples, presumably because of the consumption of FXIIIa in the clotting process (Fig. 3A). Detection of a-subunit in monomeric form requires complex activation; in plasma, this process is blocked because of the presence of heparin. In our experiment the a-subunit of FXIIIa, which represents the active enzyme, was detected in the plasma of fish but not of rats. The 160 kDa in rat plasma represent the a₂ dimer in non-active state. Following activation of the blood-clotting components the a₂ dimer form is no longer detectable as in the serum. The different intensity of the immunoreactive bands between rat and fish plasma seems to be a result of antibody specificity. These results suggest that the 55 kDa protein is the main form of the a-subunit of FXIIIa in fish plasma. They further imply that if the 55 kDa protein is a cleavage product of an 80 kDa precursor, its formation occurs immediately upon its production and/or secretion.

Cross-linking activity characteristic to FXIIIa was examined in the serum and plasma of fish and rats. Under physiological conditions obtained by the addition of heparin, hardly any cross-linking activity could be detected in rat plasma. Activity levels in rat serum, which is thought to contain only very low levels of blood-clotting components, was, as expected, even lower. In contrast, high activity was observed in fish plasma (Fig. 3B). The addition of thrombin, known to activate FXIIIa, led to a 10-fold increase in the enzyme's activity in rat plasma (Fig. 4A), while the increase in fish plasma was only 3-fold (Fig. 4B). This finding suggests that the lower enzyme activity in rat plasma in the absence of added thrombin is attributable to the presence of the enzyme in an inactive form, which might not be the case in fish (Fig. 4). Interestingly, the addition of thrombin did not cause any elevation of activity in medium conditioned by rat sciatic nerve, suggesting that in this medium either thrombin is present or the enzyme exist in a form, which unlike in the plasma, does not need further activation with thrombin (Fig. 4). The above findings suggest that the activity of FXIIIa is regulated differently in fish and rats.

### Example 4. FXIII expression following injuries of the nervous system

The differences in form and regulation of FXIII observed between fish and rat plasma prompted us to examine the amount and activity of FXIIIa in the nervous system in response to injury. Conditioned media from rat sciatic and optic nerves were prepared as described in Materials and Methods, and this was followed by Western blot analysis for detection of the a-subunit of FXIIIa. As shown in Figure 5A, a-subunit immunoreactive protein was detected in the conditioned media of both optic and sciatic nerves. Its molecular size in both preparations was similar to that of FXIIIa found in mammalian plasma, i.e. 80 kDa (rather than 55 kDa as in the fish optic nerve). Interestingly, the amount of enzyme in the rat sciatic nerve was significantly higher than in the rat optic nerve. Varying the incubation period between 1.5 and 4 h did not affect the amounts of the enzyme in the conditioned media (Fig. 5A).

To rule out the possibility that the immunoreactivity of the FXIIIa a-subunit observed in the rat nerve preparations is due to cross-reactivity with tissue-type TGase, known for its partial homology with the a-subunit of FXIIIa, we used rabbit polyclonal antibodies specific for rat tissue-type TGase. As shown in Figure 5B, antibodies raised against the a-subunit of FXIIIa did not cross-react with tissue-type TGase, nor did antibodies to tissue-type TGase cross-react with the a-subunit of FXIIIa. Moreover, the antibodies recognized the enzyme in rat nerve-conditioned medium and plasma, but not in the serum, with molecular weight similar to that obtained in human plasma, further suggesting the specificity of the antibodies to the a-subunit of FXIIIa (Fig. 5C).

### Example 5

To determine whether the FXIIIa found in the nervous tissue is derived exclusively from the plasma or, at least in part, produced locally by the nervous tissue itself, we performed Western blot analysis using antibodies directed against b-subunit of FXIII, in addition to those directed against a-subunit of FXIIIa itself. The b-subunit is known to be associated with FXIII through the link between 2 homodimers, a₂ and b₂. If all of the FXIIIa found in the nerve is plasma-derived, the ratio between the a₂ and b₂ subunits in the nerve should be the same as in the plasma. We found that the immunoreactivity of the a-subunit decreased following injury, with the sharpest drop occurring on day 1 (Fig.6A), the day on which the amount of the b-subunit was highest (Fig.6B). It therefore seems likely that FXIIIa is, at least partially, a product of the nerve tissue.

### Example 6

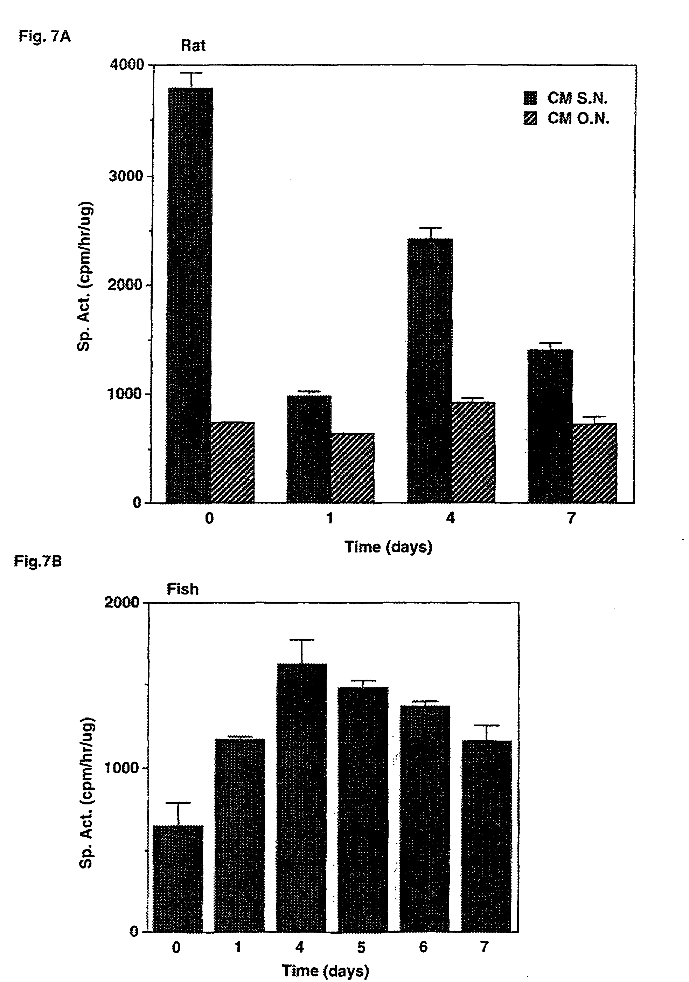

To further examine whether possible injury-induced variations in nerve-derived FXIIIa may be related to the nerve's ability to regenerate, we compared the activity of FXIIIa in regenerating and non-regenerating nerves following axonal injury. Analysis revealed that cross-linking activities were higher in medium conditioned by noninjured sciatic nerves than in medium conditioned by noninjured optic nerves of mammals. Immediately following the injury there was a sharp decrease in the enzyme's activity. Towards day 4 postinjury, when FXIIIa activity in the sciatic nerve was higher than on day 1, the activity in the rat optic nerve was still low (Fig. 7A). This difference in FXIIIa levels between mammalian CNS and PNS nerve tissue, during the first few days after injury, may be related to tissue's commitment either to recovery or to degeneration. Good correlation was found between the immunoreactivities and the enzyme cross-linking activities of the two nerve preparations at all time points tested after injury (Fig. 7A). In fish optic nerve the cross-linking activity was elevated after injury (Fig. 7B). Interestingly, these 2 preparations showed similar postinjury behavior from day 1 on, and they differed only with respect to non-injured values. This difference might be a reflection of the mode of activation of FXIIIa in fish and rat.

### Example 7. Use of human Factor XIIIa for healing of Transected Optic Nerve

Adult male SPD rats, 8-10 weeks old, average weight 300g, are deeply anesthetized (xylazine 5 mg/kg and ketamine 35 mg/kg) and their left optic nerves are exposed. Through a small opening in the meninges, the nerve fibers are completely transected 2-3 mm from the globe, without damage to the nerve vasculature and with minimal damage to the meninges. Immediately after optic nerve transection, 2 µl of Factor XIIIa are applied at the site of transection using a specially designed glass micropipet. In control animals, 2 µl of PBS are applied to the injury site.

To assay for axonal regeneration, retrograde double labeling of retinal ganglion cells was performed as follows: Crystals of the fluorescent neurotracer dye 4-Di-10-Asp (Molecular Probes, Netherlands) were applied to the transection site at the time of transection and Factor XIIIa application. After 10-12 weeks, Fluoro-Gold (Fluorochrome) (1 mg/ml in PBS) was applied 2 mm distally to the site of transection. Retinas were excised 4 days after application of the second dye and examined under a fluorescence microscope using two filters, fluorescein for visualization of the 4-Di-10-Asp and UV for the Fluoro-Gold. By double-labeling the cell bodies in this way, the possibility of mistaking axons that had escaped transection for regrowing axons is avoided. The first dye, which is applied to the transected nerve at the site of injury immediately after transection, is taken up by the cut axons and labels retinal ganglion cells of the cut axons. This means that cell bodies that are labeled by the first and second dye applications are of regrowing axons that are being cut for the second time (i.e., the first cut being the lesion itself and the second cut, made distally to it, in order to apply the dye). It should be noted that the dye-transporting capacity of regrowing axons might differ from that of intact axons cut for the first time. In addition, as it is likely that some of the retinal ganglion cells die during the period between the application of the second dye and excision of the retina, the counted double-labeled cells should be considered as the minimum number of regrowing axons. It should be noted that each dye has its own staining efficiency and therefore the different labeling efficiencies must also be taken into account.

### REFERENCES

1. DeWitt, D.A., Richey, P.L., Praprotnik, D., Silver, J. & Perry, G. (1994) Chondroitin sulfate proteoglycans are a common component of neuronal inclusions and astrocytic reaction in neurodegenerative diseases. *Brain-Res* **656**, 205-9.
2. Savio, T. & Schwab, M.E. (1989) Rat CNS white matter, but not gray matter, is nonpermissive for neuronal cell adhesion and fiber outgrowth. *J-Neurosci* **9**, 1126-33.
3. Bazan, N.G., Rodriguez de Turco, E.B. & Allan, G. (1995) Mediators of injury in neurotrauma: intracellular signal transduction and gene expression. *J-Neurotrauma* **12**, 791-814.
4. Liu, D., Yang, R., Yan, X. & McAdoo, D.J. (1994) Hydroxyl radicals generated in vivo kill neurons in the rat spinal cord: electrophysiological, histological, and neurochemical results. *J-Neurochem* **62**, 37-44.
5. So, K. & Aguayo, A. (1985) Lengthy regrowth of cut axons from ganglion cells after peripheral nerve transplantation into the retina of adult rats. *Brain Res* **328**, 349-354.
6. Bregman, B. S., Kunkel-Bagden, E., Schnell, L., Dai, D. G. & Schwab, M. E. (1995) Recovery from spinal cord injury mediated by antibodies to neurite growth inhibitors. *Nature* **378**, 498-501.
7. Cheng, H., Cao, Y. & Olson, L. (1996) Spinal cord repair in adult paraplegic rats: partial restoration of hind limb function. *Science* **273**, 510-3.
8. Lazarov-Spiegler, O., *et al.* (1996) Transplantation of activated macrophages overcomes central nervous system regrowth failure. *FASEB-J* **10**, 1296-302.
9. Schwartz, M., Hirschberg, D.L. & Beserman, P. (1995) Central Nervous System Regeneration and the Immune System. *Molecular Medicine Today* **1,** 61.
10. Perry, V.H. & Brown, M.C. (1992) Role of macrophages in peripheral nerve degeneration and repair. *Bioessays* **14,** 401-6.
11. Perry, V.H., Brown, M.C. & Gordon, S. (1987) The macrophage response to central and peripheral nerve injury. *J-Exp-Med* **165,** 1218-1223.
12. Eitan, S., *et al.* (1992) Identification of an interleukin 2-like substance as a factor cytotoxic to oligodendrocytes and associated with central nervous system regeneration. *Proc-Natl-Acad-Sci-U-S-A* **89**, 5442-6.
13. Eitan, S. & Schwartz, M. (1993) A transglutaminase that converts interleukin-2 into a factor cytotoxic to oligodendrocytes. *Science* **261**, 106-108.
14. Lorand, L., Credo, R.B. & Janus, T.J. (1981) Factor XIII (fibrin-stabilizing factor). *Methods Enzymol* **80**, 333-41.
15. Mann, K.G. & Lorand, L. (1993) Introduction: blood coagulation. *Methods Enzymol* **222,** 1-10.
16. Muszbek, L., Adany, R. & Mikkola, H. (1996) Novel aspects of blood coagulation factor XIII. I. Structure, distribution, activation, and function. *Crit-Rev-Clin-Lab-Sci* **33**, 357-421.
17. Greenberg, C.S., Birckbichler, P.J. & Rice, R.H. (1991) Transglutaminases: multifunctional cross-linking enzymes that stabilize tissues. *FASEB-J* **5,** 3071-7.
18. Toida, M., *et al.* (1995) Characterization of cells containing factor XIII subunit a in benign and malignant buccal lesions. *Histochem-J* **27,** 449-56.
19. Toida, M., Oka, N., Takami, T. & Adany, R. (1995) Accumulation of cells containing factor XIII subunit a around the foci of intense fibrosis in human epulides. *Histochem-J* **27,** 440-8.
20. Adany, R. (1996) Intracellular factor XIII: cellular distribution of factor XIII subunit a in humans. *Semin-Thromb-Hemost* **22,** 399-408.
21. Lai, T.S., Santiago, M.A., Achyuthan, K.E. & Greenberg, C.S. (1994) Purification and characterization of recombinant human coagulant factor XIII A-chains expressed in E. coli. *Protein-Expr-Purif* **5,** 125-32.
22. Muszbek, L., Adany, R., Szegedi, G., Polgar, J. & Kavai, M. (1985) Factor XIII of blood coagulation in human monocytes. *Thromb-Res* **37**, 401-10.
23. Kiesselbach, T.H. & Wagener, R.H. (1972) Demonstration of factor XIII in human megacaryocytes by a fluorescent antibody technique. *Ann. N.Y. Acad. Sci.* **202**, 318-28,.
24. Adany, R. & Antal, M. (1996) Three different cell types can synthesize factor XIII subunit A in the human liver. *Thromb-Haemost* **76,** 74-9.
25. Pike, C.J., Vaughan, P.J., Cunningham, D.D. & Cotman, C.W. (1996) Thrombin attenuates neuronal cell death and modulates astrocyte reactivity induced by beta-amyloid in vitro. *J-Neurochem* **66,** 1374-82.
26. Vaughan, P.J., Pike, C.J., Cotman, C.W. & Cunningham, D.D. (1995) Thrombin receptor activation protects neurons and astrocytes from cell death produced by environmental insults. *J-Neurosci* **15**, 5389-401.
27. Vaughan, P.J. & Cunningham, D.D. (1993) Regulation of protease nexin-1 synthesis and secretion in cultured brain cells by injury-related factors. *J-Biol-Chem* **268**, 3720-7.
28. Guttridge, D.C., Lau, A.L. & Cunningham, D.D. (1993) Protease nexin-1, a thrombin inhibitor, is regulated by interleukin-1 and dexamethasone in normal human fibroblasts. *J-Biol-Chem* **268,** 18966-74.
29. Eitan, S., *et al.* (1994) Recovery of visual response of injured adult rat optic nerves treated with transglutaminase. *Science* **264**, 1764-1768.

## Claims

1. Use of Factor XIIIa for the preparation of a pharmaceutical composition for nerve healing.

2. The use according to Claim 1, wherein the pharmaceutical composition is in a form for local administration at the site of injury or disease.

## Patentansprüche

1. Verwendung von Faktor XIIIa für die Herstellung eines Arzneimittels zur Nervenheilung.

2. Verwendung nach Anspruch 1, wobei das Arzneimittel in einer Form ist, die zur lokalen Verabreichung an der Stelle der Verletzung oder Krankheit geeignet ist.

## Revendications

1. Utilisation du Facteur XIIIA pour la préparation d'une composition pharmaceutique pour la régénération nerveuse.

2. Utilisation selon la revendication 1, où la composition pharmaceutique est dans une forme pour l'administration locale dans le site de lésion ou maladie.
